(19) Europäisches Patentamt
European Patent Office
Office européen des brevets

(11) Publication number: **0 432 297 A1**

# (12) EUROPEAN PATENT APPLICATION

(21) Application number: **89123016.1**

(51) Int. Cl.5 **C07D 501/36**

(22) Date of filing: **13.12.89**

(43) Date of publication of application:
**19.06.91 Bulletin 91/25**

(84) Designated Contracting States:
**BE CH DE ES FR GR IT LI**

(71) Applicant: **TECHNOLOGITSCHEN KOMBINAT SA PROMISCHLENA MIKROBIOLOGIA**

**Rasgrad(BG)**

(72) Inventor: **Dobreva, Nevena Dimitrova**
**Tonev Strasse 39a-A**
**1618 Sofia(BG)**
Inventor: **Kovatscheva, Miloslava Dineva**
**Boul. Vl. Saimov 38-B**
**Sofia(BG)**
Inventor: **Makedonska, Vanja Borissova**
**N. Mirtschev Strasse 27-4**
**1156 Sofia(BG)**
Inventor: **Vassileva, Blagina Kresteva**
**V. Grigorovitsch Strasse 12**
**1606 Sofia(BG)**
Inventor: **Georgiev, Alexander Hristov**
**Boul. Botevgradsko Chaussée Block 14-A**
**Sofia(BG)**
Inventor: **Manov, Hristo Dimitrov**
**G. Dimitrov Strasse 36-A**
**7200 Rasgrad(BG)**
Inventor: **Dekov, Vladimir Stamenov**
**Boul. Kiril i Metodi 4**
**7200 Rasgrad(BG)**
Inventor: **Papasova, Penka Georgieva**
**T. Petrov Strasse 7**
**1113 Sofia(BG)**
Inventor: **Radoslavov, Ivan Hristov**
**Komplex Borovo Block 25**
**Sofia(BG)**

(74) Representative: **von Füner, Alexander, Dr. et al**
**Patentanwälte v. Füner, Ebbinghaus, Finck**
**Mariahilfplatz 2 & 3**
**W-8000 München 90(DE)**

## (54) A method for the preparation of the sodium salt of O-formyl cefamandole.

(57) This invention refers to a method for the preparation of the broad spectrum antibiotic, sodium cefamandole naphthate.

The object is to provide a technological method warranting a high yield, high purity and stability of the product.

The method comprises the following stages:
silylation of the initial product 7-amino-3-(1-methyl-1H-tetrazol-5-yl-thiomethyl)-3-cephem-4-carboxylic acid, acylation and then desilylation. In the method of this invention the desilylation is accompanied by the removal of the solvent. The obtained product is further dissolved in ethylacetate, decolourized, the solvent is removed and the obtained oily product is dissolved in dry methylene chloride, crystallized, separated and dissolved in a non-aqueous medium of two organic solvents and precipitated as sodium salt with sodium acetate or sodium-2-ethylhexanoate.

EP 0 432 297 A1

# A METHOD FOR THE PREPARATION OF THE SODIUM SALT OF O-FORMYL CEFAMANDOLE

This invention refers to a method for the preparation of the sodium salt of O-formyl cefamandole or the sodium salt of 7-(D-2-formyloxy-2-phenylacetamido)-3-(1-methyl-1H-tetrazol-5-yl-thiomethyl)-3-cephem-4-carboxylic acid, known as sodium cefamandole naphthate, a broad spectrum antibiotic for parenteral administration.

There is a method for the preparation of the sodium salt of O-formyl cefamandole-gamma-form by means of a silylation of 7-amino-3-(1-methyl-1H-tetrazol-5-yl-thiomethyl)-3-cephem-4-carboxylic acid (7-AMTCA) with hexamethyldisilazane (HMDS), acylation of the so-obtained N,O-bis trimethylsilyl ester of 7-AMTCA with D-(-)-2-formyloxy-2-phenylacetyl chloride, desilylation and preparation of O-formyl cefamandole sodium salt, without or with the isolation of O-formyl cefamandole acid (O-FCM acid) with a yield of O-formyl cefamandole sodium salt of 84.5% on the basis of 7-AMTCA (Bulgarian author's certificate No. 36849).

A disadvantage of this method is the fact that the sodium salt of O-formyl cefamandole is obtained with lower stability regarding the requirements towards the solubility and the colour of its aqueous solutions during storage, which is due to the lower stability of O-formyl cefamandole acid.

The object of this invention is to provide a technological method for the preparation of O-formyl cefamandole sodium salt with a high yield, high purity and stability.

The method of this invention can be presented by the following chemical scheme:

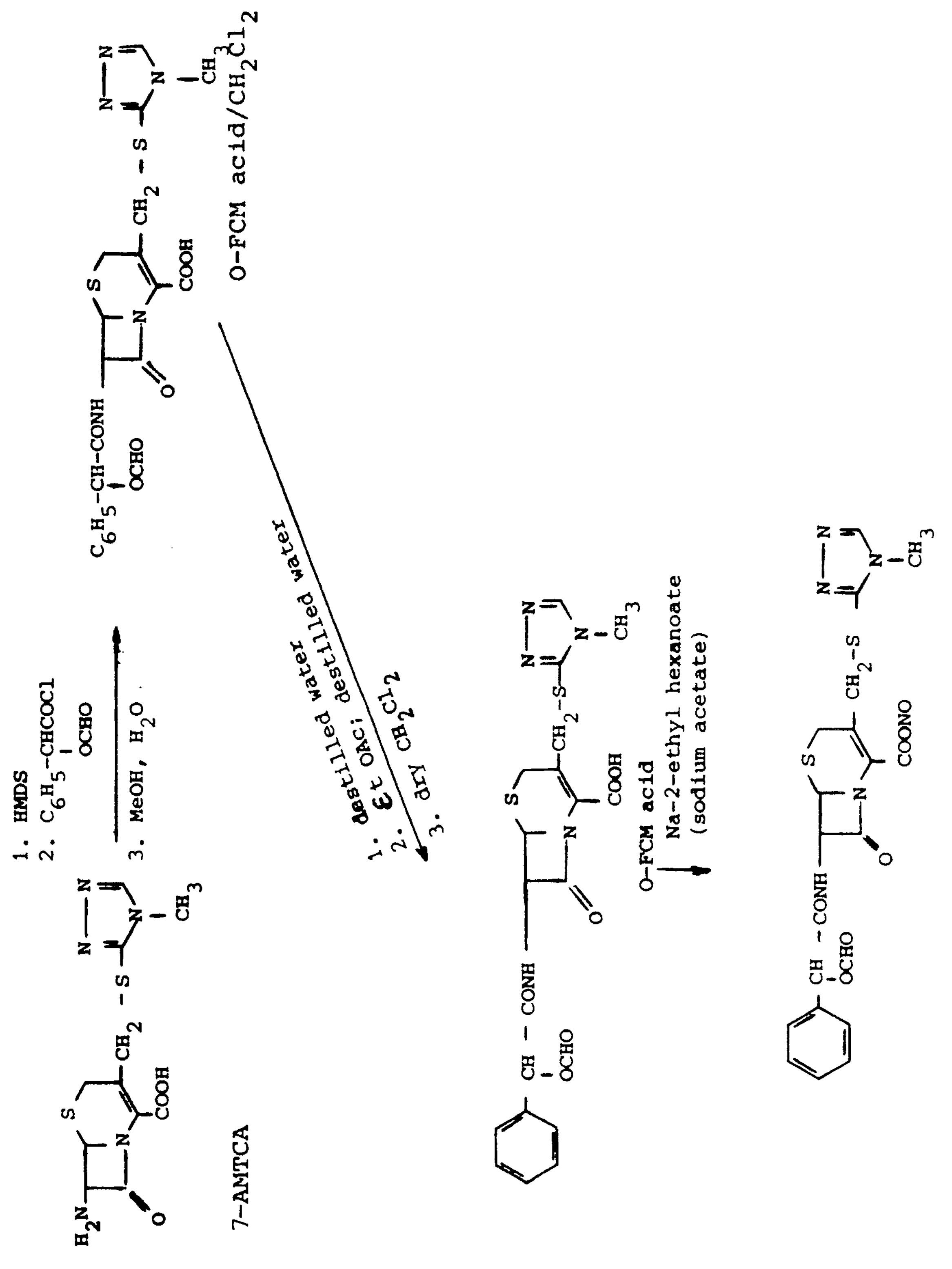


The method of this invention is as follows: from the O-formyl cefamandole acid, obtained from 7-AMTCA through silylation with hexamethyldisilazane, acylation with D-(-)-2-formyloxy-2-phenyl-acetylchloride and after the desilylation, the solvent is removed; then the obtained product is dissolved in ethylacetate, decolourized, then the ethylacetate is removed till an oily product is obtained, which is dissolved in dry methylene chloride, then crystallized through cooling, isolated and dissolved in non-aqueous medium consisting of two organic solvents in a volume ratio from 1:0.8 to 1.5:1.2; then it is precipitated as a sodium salt with sodium-2-ethylhexanoate or sodium acetate at room temperature.

The solvent methylene chloride is removed from the O-formyl cefamandole acid through vacuum-distillation at a temperature of 10-20 °C till its complete removal. The product is dissolved in ethylacetate and is decolourized with charcoal at 20-30 °C. The obtained oily product is dissolved in dry methylene chloride and crystallized at cooling.

The obtained crystalline O-formyl cefamandole acid is precipitated as sodium salt in non-aqueous organic medium consisting of acetonitrile/isopropanol, acetone/isopropanol or methanol/isopropanol with 2-ethylhexanoate or sodium acetate.

The advantages of the proposed method are the obtaining of crystalline O-formyl cefamandole acid with high stability and high purity as well as the obtaining of its sodium salt with a high yield of 87-90%, high purity and stability.

The method as per this invention provides for the obtaining of a sodium salt of O-formyl cefamandole, corresponding to the requirements of U.S. Pharmacopoeia XXI.

This invention can be illustrated through the following examples:

Example 1: Preparation of O-formyl cefamandole

15 g (0.046 moles) 7-AMTCA are suspended in 180 ml dry methylene chloride; then 9.6 ml (0.046 moles) hexamethyldisilazane are added. The suspension is stirred at 40 °C till its dissolution; then it is cooled to -10 °C and 6.0 ml (0.046 moles) N,N-dimethylaniline and 7.8 ml (0.046 moles) D-(-)- 2-formyloxy-2-phenylacetyl chloride are added. The reaction mixture is agitated for one hour at 0 °C and for one hour at 5 °C. Then 50 ml methanol and 100 ml water are added; after 20 minutes of agitation the mixture is stratified. The methylene chloride solution of the O-formyl cefamandole acid is subjected to vacuum-distillation at 15 °C till the removal of the methylene chloride. Then 150 ml ethylacetate are added and the solution is decolourized with 0.5 g charcoal at 5 °C. The suspension is filtered and the filtrate is subjected to vacuum-distillation at 22 °C till an oily material is obtained; then 100 ml of dry methylene chloride are added and the acid crystallizes out during agitation and cooling to 5 °C. The suspension is agitated for 2 hours at the same temperature, filtered, washed with cold methylene chloride and dried at 40 °C under vacuum. 20.2 g of O-formyl cefamandole are obtained (90% yield) with a purity of 98%; m.p. 128-130 °C.

Example 2: Preparation of O-formyl cefamandole sodium salt

To a solution of 5.0 g (0.010 moles) O-formyl cefamandole acid in 80 ml non-aqueous mixture of acetonitrile/isopropanol (1.2:1) at stirring is added a solution of 1.66 g (0.010 moles) of sodium-2-ethyl hexanoate in a mixture of acetonitrile/isopropanol (1.2:1.0). After agitating for 2 hours at 22 °C the separated precipitate is filtered, washed with 10 ml of acetonitrile/isopropanol (1.2:1.0) and dried under vacuum at 45 °C. 0.05 g of sodium O-formyl cefamandole acid are obtained, (i.e. a yield of 96.7% towards O-formyl cefamandole acid or 87.03% towards 7-AMTCA) with a purity of 98%; m.p. 191-192 °C.

NMR: $D_2O$,δ,ppm: 3.40(d,2H), 3.90(S,>$NCH_3$), 4-20(S,-$CH_2$-S), 4.90(d,H-6), 5.55(d,H-7),

6.13(S,-CH-CO),
        |
        O
        |

7.45(m,$C_6H_5$), 8.35(S,-CHO)

Example 3: Preparation of O-formyl cefamandole sodium salt

To a solution of 20 g (0.04 moles) O-formyl cefamandole acid in 260 ml non-aqueous mixture of acetonitrile/isopropanol (1.2:1) at stirring is added a solution of 6.8 g (0.04 moles) sodium-2-ethyl hexanoate in a mixture of 20 ml. After agitating the separated precipitate is filtered, washed with a mixture of acetone/isopropanol (1.2:1.0) and dried under vacuum at 45 °C. 20.4 g of sodium O-cefamandole salt are obtained (i.e. a yield of 97.6% towards O-cefamandole acid or 87.8% towards 7-AMTCA) with a purity of 97%; m.p. 192 °C.

The NMR data are the same as for Example 2.

Example 4: The preparation of O-formyl cefamandole sodium salt

To a solution of 10 g (0.02 moles) O-formyl cefamandole acid in 100 ml dried methanol at stirring a

solution of 1.65 g (0.02 moles) non-aqueous sodium acetate in methanol is added. Then 80 ml dried isopropanol is added. After agitating the separated precipitate is filtered, washed with a mixture of methanol/isopropanol (1.2:1.0) and dried under vacuum at 45° C. 9.85 g of sodium O-formyl cefamandole are obtained, i.e. a yield of 94.4% with a purity of 97%; m.p. 191-192° C.

The NMR data are the same as for Example 2.

## Claims

1. A method for the preparation of the sodium salt of O-cefamandole or 7-(-2-formyloxy-2-phenylacetamido)-3-(1-methyl-1H-tetrazol-5-yl-thiomethyl)-3-cephem-4-carboxylic acid from 7-amino-3-(1-methyl-1H-tetrazol-5-yl-thiomethyl)-3-cephem-4-carboxylic acid through a silylation with hexamethyldisilazane, acylation with D-(-)-2-formyloxy-2-phenylacetyl chloride and desilylation,
**characterized in that** the solvent is removed from the obtained O-formyl cefamandole after desilylation, the obtained product is dissolved in ethylacetate, decolourized, the ethylacetate is removed till an oily product is obtained, which is dissolved in dry methylene chloride and crystallized at cooling, then is separated and dissolved in a non-aqueous medium of two organic solvents in a volume ratio from 1:0.8 to 1.5:1.2 and precipitated as sodium salt with sodium-2-ethylhexanoate or sodium acetate at room temperature.

2. A method according to Claim 1, **characterized in that** the methylene chloride solvent is removed by vacuum-destillation at 10-20° C.

3. A method according to Claim 1, **characterized in that** the decolourization is carried out with charcoal at a cooling to O-5° C, and the ethylacetate is removed by vacuum-destillation at 20-30° C.

4. A method according to Claim 1, **characterized in that** the non-aqueous organic medium can be acetone/isopropanol, acetonitrile/isopropanol or methanol/isopropanol.

European Patent Office

# EUROPEAN SEARCH REPORT

Application Number

EP 89 12 3016

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | FR-A-2 306 698 (ELI LILLY)<br>* Complete specification *<br>--- | 1 | C 07 D 501/36 |
| A | US-A-4 168 376 (ELI LILLY)<br>* Complete specification *<br>--- | 1 | |
| D,A | BG-A- 36 849 (FARMAHIM, SOFIA)<br>* Complete specification *<br>----- | 1 | |
| | | | TECHNICAL FIELDS SEARCHED (Int. Cl.5) |
| | | | C 07 D 501/00 |

The present search report has been drawn up for all claims

| Place of search | Date of completion of the search | Examiner |
|---|---|---|
| THE HAGUE | 02-08-1990 | LUYTEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)